# EUROPEAN PATENT APPLICATION

(11) **EP 4 195 214 A1**
(43) Date of publication of application: **14.06.2023**
(21) Application number: 22160516.5
(22) Date of filing: 07.03.2022
(51) Int. Cl.: G16H 30/20, G16H 30/40

(54) **DISPLAYING RELEVANT PRIOR REPORTS IN A TELEMEDICINE SETTING**

(30) Priority: 13.12.2021 US 202163288671 P
(71) Applicant: Koninklijke Philips N.V., 5656 AG Eindhoven (NL)
(72) Inventor: MABOTUWANA, Thusitha Dananjaya De Silva, Eindhoven (NL); BUB, Lawrence, Eindhoven (NL); WAKELY, Jesse, Eindhoven (NL)
(74) Representative: Philips Intellectual Property & Standards

(57) **Abstract**

A non-transitory computer readable medium (26) stores instructions executable by at least one electronic processor (20) to a method (100) of selecting relevant prior reports in telemedicine. The method includes receiving a query for prior reports (34); selecting one or more prior reports; reformatting at least one of the selected prior reports; and transmitting the re-formatted prior reports to an electronic processing device (18) operable by a medical professional.

## Description

### FIELD OF THE IVENTION

The following relates generally to the radiology arts, radiology reporting arts, telemedicine arts, radiology examination reading arts, and related arts

### BACKGROUND OF THE INVENTION

Picture Archiving and Communication System (PACS) and Radiology Information System (RIS) databases are typically designed for use within large healthcare environments (such as hospitals, academic medical centers and integrated delivery networks) where the radiologists are also usually onsite, employed, and contracted directly by the facility and interpret while being connected to the same electronic information technology (IT) network within which images are acquired and stored in the PACS. Diagnostic interpretation can be performed while off-site as well (e.g., from the radiologist's home), but the radiologists is required still to be connected to the hospital network via virtual private network (VPN), and often restricted to using the same PACS system used in the hospital.

Teleradiology is a different environment. In teleradiology, a hospital or other radiology laboratory sends images to an external entity (the teleradiology provider) via the Internet, and a radiologist employed by the teleradiology provider reads the imaging examination, prepares a radiology report presenting the findings, and that radiology report is returned to the hospital. In a typical teleradiology setting, the images that need to be interpreted are uploaded via the internet to a separate RIS/PACS system managed by the external teleradiology provider. In this setting, the radiologist interpreting the medical images is not physically present in the location where the images were generated, is not on the hospital IT network, and may not be using the same PACS system. As such, the teleradiologist does not have access to any prior imaging studies on the hospital PACS that might be useful in interpreting the current imaging study. The images and other metadata of each imaging study (current and any prior studies) is typically stored as a package in a digital imaging and communications in medicine (DICOM) format, and hence the images are transmitted over the internet to a teleradiology provider's system in DICOM format. Such transmission can be very costly in terms of bandwidth, time-consuming and also not always needed for accurate diagnostic interpretation. It also may not be immediately apparent to the person outsourcing a current imaging study to the teleradiology provider as to which prior studies, if any, are available and relevant. As a result, only the acquired images of the current study are usually uploaded, and not the images of any prior studies.

However, when these prior studies are needed, support staff or the interpreting radiologist needs to manually request these from the acquiring site. Oftentimes, it may not be obvious if prior studies are even available, and the radiologist may interpret the current study on its own due to this uncertainty and the time-consuming nature of gaining access to the priors which can possibly adversely affect patient care.

The following discloses certain improvements to overcome these problems and others.

### SUMMARY OF THE INVENTION

In some embodiments disclosed herein, a non-transitory computer readable medium stores instructions executable by at least one electronic processor to a method of selecting relevant prior reports in telemedicine. The method includes receiving a query for prior reports; selecting one or more prior reports; re-formatting at least one of the selected prior reports; and transmitting the re-formatted prior reports to an electronic processing device operable by a medical professional.

In some embodiments disclosed herein, a non-transitory computer readable medium stores instructions executable by at least one electronic processor to a method of selecting relevant prior reports in telemedicine. The method includes selecting one or more prior reports; displaying the selected prior reports on an electronic processing device operable by a support professional; receiving one or more inputs, provided by the support professional, indicative of an approval of one or more of the selected prior reports; and converting text of the selected prior reports to a digital imaging and communications in medicine (DICOM) image format.

In some embodiments disclosed herein, a method of selecting relevant prior reports in telemedicine includes determining one or more prior reports related to a current medical imaging examination to be read; retrieving the one or more prior reports from a Picture Archiving and Communication System (PACS) database; re-formatting the selected prior reports from text to DICOM image representations of the respective selected prior reports; inserting the DICOM image representations of the respective selected prior reports into a DICOM representation of the current medical imaging examination to be read as new series to generate an enhanced DICOM representation of the current medical imaging examination to be read; transmitting the enhanced DICOM representation of the current medical imaging examination to be read to an electronic processing device operable by a medical professional; and displaying, on the electronic processing device operable by a medical professional, the re-formatted prior reports.

One advantage resides in providing a tool for selecting prior radiology reports for viewing during a current radiology reading session.

Another advantage resides in automatically selecting prior radiology reports for viewing during a current radiology reading session.

Another advantage resides in improving efficiency and speed of radiology reading sessions.

Another advantage resides in improvements to a radiology department information technology (IT) infrastructure and to a teleradiologist IT infrastructure to improve radiology reading sessions.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
Fig. 1 diagrammatically illustrates an apparatus for selecting relevant prior reports in telemedicine in accordance with the present disclosure.
Fig. 2 diagrammatically illustrates example flow chart operations performed by the apparatus of Fig. 1.
Fig. 3 diagrammatically illustrates an example of hospital and teleradiology IT systems with automated prior study retrieval.
Fig. 4 diagrammatically shows a portion of the systems of Fig. 3 in conjunction with processing steps to fetch relevant prior images and reports and insert into teleradiology PACS.
Fig. 5 shows an example prior reports used by the apparatus of Fig. 1.

### DETAILED DESCRIPTION OF EMBODIMENTS

Medical facilities can provide remote radiology readings, sometimes referred to as teleradiology. For complete reading of many radiology examinations, the radiologist may need to review prior radiology examinations of the patient. For example, a radiology examination for monitoring a malignant tumor benefits from comparison of the imaged tumor in the currently read examination with tumor size assessed in a prior examination of the same tumor, for example performed before chemotherapy or radiation therapy treatment of the tumor. Furthermore, the radiology examination reading may benefit by having the radiologist review non-radiology reports on the patient.

In the case of teleradiology, the file size of the imaging examination digital imaging and communications in medicine (DICOM) records is large, and transmission from the hospital to the teleradiologist consumes substantial bandwidth over the hospital network and Internet. In view of this, and since it may not be apparent which (if any) prior studies are relevant, the usual practice is for the teleradiologist to receive only the current radiology examination which is to be read. The teleradiologist (or radiology support staff preparing the images for interpretation) must then proactively request any relevant prior radiology (or other) examinations. However, the radiologist (or support staff) may be unaware of these prior examinations, or may neglect to request them due to time constraints. Moreover, the DICOM link between the hospital and teleradiologist generally supports transmission of DICOM data, which is designed for storage and transfer of images and may not support a wide range of examination report formats.

To address these difficulties, the following discloses modifications to the hospital radiology department information technology (IT) infrastructure and to the teleradiologist IT infrastructure. On the hospital IT side, a Picture Archiving and Communication System (PACS) gateway and automation engine is provided, which provides an interface for directing the hospital PACS to transfer imaging examinations to the teleradiologist. This may also optionally include an HL7 feed to transfer non-radiology examination reports to the teleradiologist. To enable text-based reports to be transmitted via the DICOM link, these text reports are converted to DICOM-compatible images that can then be handled just as any other DICOM image (where DICOM images are usually medical images, e.g. magnetic resonance imaging (MRI) images, computed tomography (CT) images, or so forth).

On the teleradiology IT side, a bi-directional prior study processor with query-retrieve functionality is implemented which identifies relevant prior studies by communicating with the onsite gateway which communicates with the onsite PACS system to determine all the studies associated with a given patient. The prior study processor then determines which prior studies should be sent to the teleradiologist. In present workflows, radiology support staff handles intake and processing of new radiology examination reading work orders. Hence, the prior examinations recommended for transfer by the prior study processor are reviewed by radiology support on a graphical user interface (GUI) before any of these examinations are actually fetched. After review and possible editing of the recommended prior examinations via the GUI (including for example editing the recommended prior examinations by converting them to a lower resolution format, and/or including only a subset of the data of the prior examination such as the written report and perhaps one, two, or a few key images so identified in the report and/or flagged in the examination as key images), the chosen prior examinations are sent to the PACS gateway to have them sent to the teleradiology IT system. Each prior examination is formatted as a new DICOM series of images (including the corresponding examination report converted to an image and optionally one or more key images, optionally converted to the lower resolution format) that is associated to the DICOM record of the current radiology examination to be read.

Finally, at the radiology workstation, the entire current radiology examination to be read, along with the associated prior examination DICOM series, is reviewed by the teleradiologist. Optionally, optical character recognition (OCR) may be applied to the images representing prior radiology (or other) reports to enable the teleradiologist to perform text word searches on those prior reports.

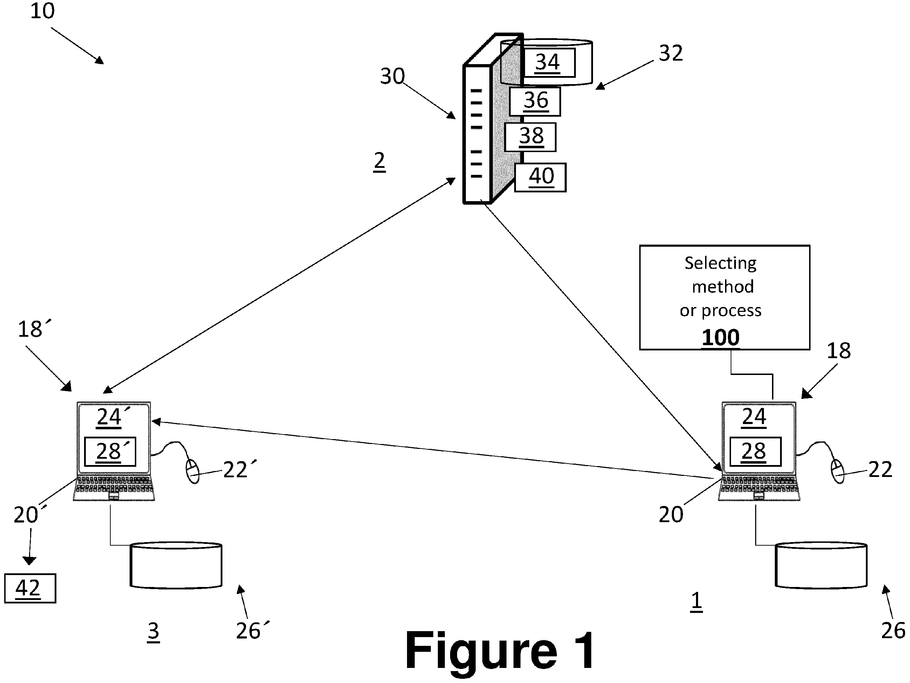

With reference to Fig. 1, an illustrative apparatus 10 for performing a radiology reading session is diagrammatically shown in a radiology reading room 1. The apparatus 10 can comprise an electronic processing device 18 operable by a medical professional (i.e., a teleradiologist). The electronic processing device 18 can comprise any suitable electronic device, such as a workstation computer, or more generally a computer. The electronic processing device 18 may also include a server computer or a plurality of server computers, e.g., interconnected to form a server cluster, cloud computing resource, or so forth, to perform more complex computational tasks. The electronic processing device 18 includes typical components, such as an electronic processor 20 (e.g., a microprocessor), at least one user input device (e.g., a mouse, a keyboard, a trackball, and/or the like) 22, and a display device 24 (e.g., an LCD display, plasma display, cathode ray tube display, and/or so forth). In some embodiments, the display device 24 can be a separate component from the electronic processing device 18, or may include two or more display devices.

The electronic processor 20 is operatively connected with one or more non-transitory storage media 26. The non-transitory storage media 26 may, by way of non-limiting illustrative example, include one or more of a magnetic disk, RAID, or other magnetic storage medium; a solid-state drive, flash drive, electronically erasable read-only memory (EEROM) or other electronic memory; an optical disk or other optical storage; various combinations thereof; or so forth; and may be for example a network storage, an internal hard drive of the workstation 18, various combinations thereof, or so forth. It is to be understood that any reference to a non-transitory medium or media 26 herein is to be broadly construed as encompassing a single medium or multiple media of the same or different types. Likewise, the electronic processor 20 may be embodied as a single electronic processor or as two or more electronic processors. The non-transitory storage media 26 stores instructions executable by the at least one electronic processor 20. The instructions include instructions to generate a visualization of a graphical user interface (GUI) 28 for display on the display device 24.

The electronic processing device 18 is also in communication with a medical facility IT department 2 that includes a server computer 30 with a PACS database 32 that stores prior reports 34 (i.e., prior radiology reports). A DICOM processor 36 is configured to convert the prior reports 34 to a DICOM image format for transfer to the electronic processing device 18. In some embodiments, a HL7 processor 38 is configured to transfer one or more non-radiology examination reports 40 to the electronic processing device 18.

The electronic processing device 18 is also in communication with a teleradiology IT department 3 that includes an electronic processing device 18' operable by a support professional (i.e., radiology support staff). The support professional electronic processing device 18' includes similar components as the teleradiology electronic processing device 18, with corresponding components denoted with a prime (') symbol (and thus will not be repeated for brevity). The support professional electronic processing device 18' includes a prior study module 42 implemented in the electronic processor 20', and is configured to identify relevant prior reports 32 for a current radiology reading session. The prior reports can be identified, for example, based on matching the anatomy of the prior study performed during a specific duration (e.g., last 3 years), or using a standard or custom ontology to determine semantic distance between anatomy concepts. Some other illustrative examples of systems and methods for selecting the prior reports are disclosed, for example, in Serlie et al., U.S. Pub. No. 2015/0379210 A1 published December 31, 2015, which is incorporated herein by reference in its entirety.

It will be appreciated that the radiology reading room 1, the medical facility IT department 2, and the teleradiology IT department 3 can be disposed in different locations in a single medical facility, across multiple medical facilities (either local or global), or any other suitable geographic arrangement.

The apparatus 10 is configured as described above to perform a method or process 100 of selecting relevant prior reports in telemedicine. The non-transitory storage medium 26, the server computer 30, and the non-transitory storage medium 26' store instructions which are readable and executable by the at least one electronic processor 20 of the teleradiology electronic processing device 18, the DICOM processor 36 of the server computer 30, and the at least one electronic processor 20' of the support professional electronic processing device 18' to perform disclosed operations including performing the method or process 100. In some examples, the method 100 may be performed at least in part by cloud processing.

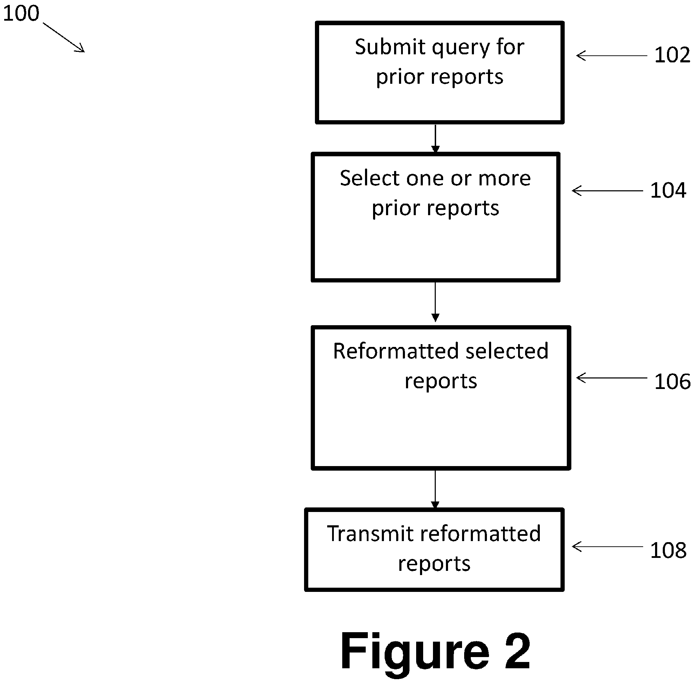

With reference to Fig. 2, and with continuing reference to Fig. 1, an illustrative embodiment of an instance of the method 100 is diagrammatically shown as a flowchart. At an operation 102, a query for prior reports 34 is submitted by the teleradiologist via the teleradiology electronic processing device 18 to the support professional electronic processing device 18'. The query is then received by the radiology report staff.

At an operation 104, the prior study module 42 is configured to select one or more prior reports 34. These selected prior reports 34 are retrieved from the PACS database 32. The selected prior reports 34 are then displayed on the display device 24' of the support professional electronic processing device 18'. The radiology report staff member then provides one or more inputs via the at least one user input device 22' indicative of an approval of the one or more of the selected prior reports 34 that are to be re-formatted for the teleradiologist.

In some embodiments, the prior report selection operation 104 includes an automatic selection and reformatting process. To do so, a link between the radiology reading room 1 and the teleradiology IT department 3 can be a two-way communication link, and upon reviewing the selected prior reports 34, the teleradiologist can automatically be re-transmitted with an alternative format. For example, the server computer 30 may find 10 relevant prior reports 34. Based on bandwidth and uncertainty about which of these prior reports the teleradiologist may want to review in detail, all 10 prior reports 34 can be sent, optionally along with one or a few associated images converted from original resolution to a reduced resolution format. Upon review, the teleradiologist can elect, for example 1 or 2 prior reports 34 for closer review (e.g., using the at least one user input device 22'), and instruct the server computer 30 to resend those designated prior reports 34 with the original resolution images.

At an operation 106, at least one of the selected (i.e., approved) prior reports 34 is reformatted by the DICOM processor 36 from text to images. To do so, the DICOM processor 36 is configured to convert text of the selected prior reports 34 to a DICOM image format. Each of the re-formatted prior reports 34 is inserted into a DICOM formatted current medical examination to be read by the medical professional as a new series inserted into the DICOM formatted current medical examination. In some example, the query for the prior reports 34 can be generated based on the DICOM formatted current medical examination.

At an operation 108, the re-formatted prior reports 34 are transmitted from the server computer 30 to the teleradiology electronic processing device 18. The re-formatted prior reports 34 are displayed on the display device 24. In some embodiments, one or more non-radiology reports 40 can be transmitted by the HL7 processor 38 to the teleradiology electronic processing device 18 for display on the display device 24. In some examples, an OCR process can be performed on the displayed re-formatted prior reports 34 for analysis by the teleradiologist.

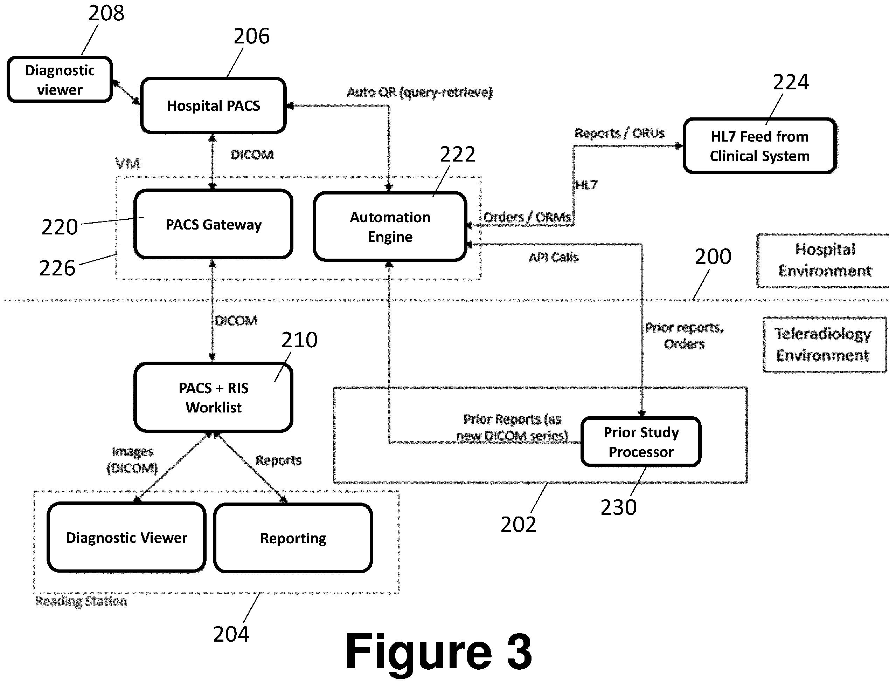

With reference to Fig. 3, an example of hospital IT system (upper portion above dividing line 200) and a teleradiology IT system (lower portion below dividing line 200) is diagrammatically shown, along with an automated prior study retrieval component 202 analogous to that just described. The teleradiologist performs readings at a reading station 204. Also shown in Fig. 3 is the hospital PACS 206, a diagnostic viewer 208 via which a hospital worker can view PACS data retrieved from the PACS 206, and a corresponding PACS 210 of the teleradiology IT system (which in the illustrative example includes a radiology information system, RIS, component). To provide the prior reports identification and retrieval capability, a site gateway 220 is provided that communicates with the onsite PACS system 206 of the hospital IT system. The overall system further includes an automation engine 222 that bi-directionally communicates with the site gateway to perform various query-retrieve operations such as getting a list of exams for a given patient. In the illustrative example of Fig. 3, the automation engine 222 is operatively connected to access the hospital PACS 206 as well as other databases of the hospital IT system via an HL7 feed 224. In the illustrative example, the site gateway 220 and automation engine 222 are implemented on the hospital IT system as a virtual machine (VM) 226. However, these components 220, 222 could be otherwise embodied, for example as an edge server owned by the teleradiology provider that is connected with the hospital IT system through suitable firewall or other IT security mechanisms. The prior study retrieval component 202 includes an intelligent study matching module 230 that determines the minimum set of prior studies that are relevant to current study and requests for a particular DICOM study from the onsite PACS. A graphical user interface (e.g., the diagnostic viewer 208) allows a user to further refine the list of prior studies that should be fetched from the onsite PACS system. The module 230 retrieves prior reports from multiple formats (e.g., HL7, CSV) via the automation engine 222 and transforms into a structured, formatted text report. These are then converted to DICOM-compatible image format and each prior study is inserted in the DICOM package for the current study as a new series.

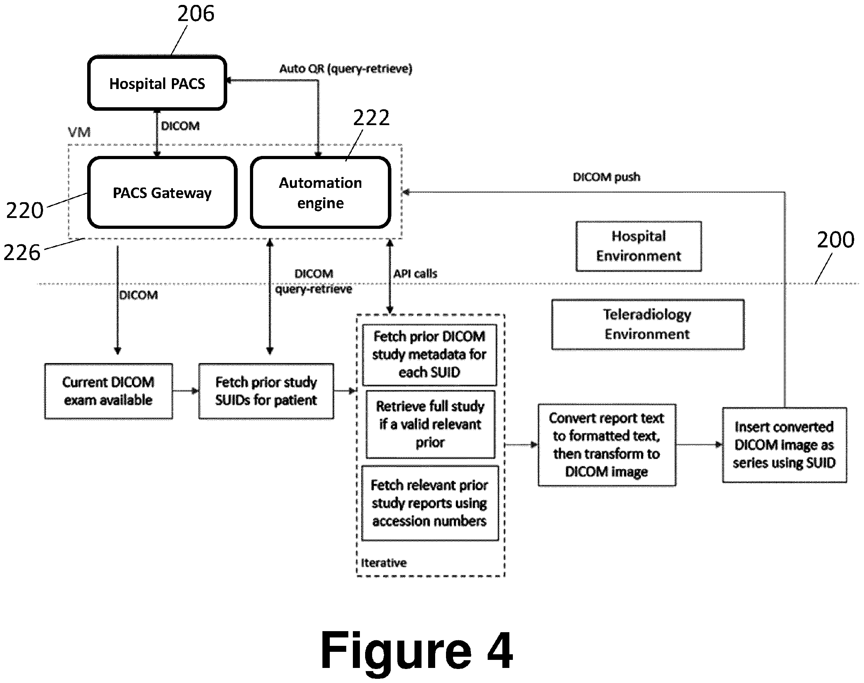

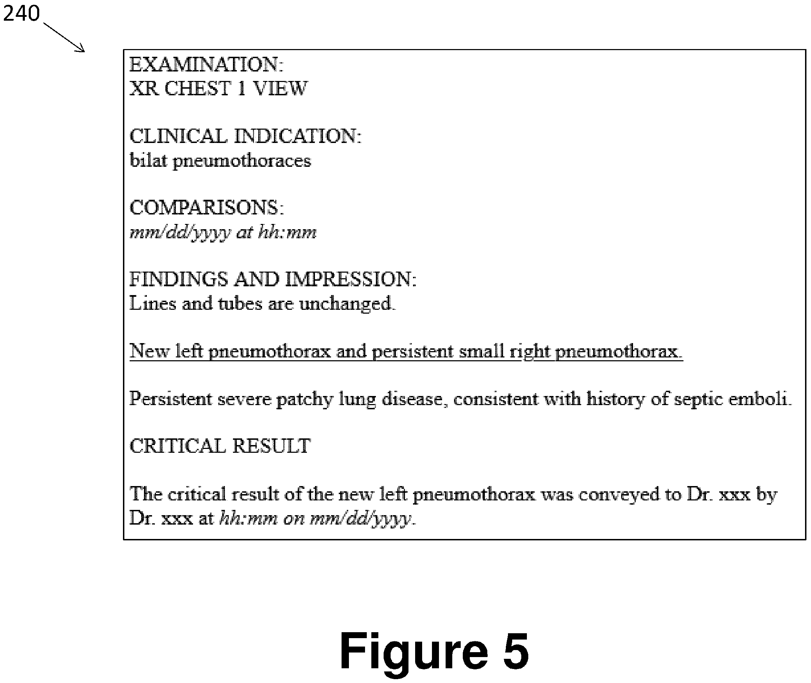

With reference to Fig. 4, a portion of the systems of Fig. 3 is again diagrammatically shown, here in conjunction with processing steps to fetch relevant prior images and reports and insert into teleradiology PACS. The software-based PACS gateway 220 that communicates with the onsite PACS system 206 is deployed on the virtual machine 226 that is hosted on the hospital IT network (or alternatively is implemented on an edge server or other IT architecture). This module 220 can request specified DICOM studies to be pushed to the teleradiology IT system. The prior study processor 202 (see Fig. 3) is an intelligent module that, in collaboration with the automation engine 222, determines the prior studies that are relevant to current study based on the current study description and DICOM metadata. There can be many approaches to determine the set of relevant prior studies, and in one approach, this could be based on matching the anatomy of the prior study performed during a specific duration (e.g., last 3 years). In another approach, a standard or custom ontology can be used to determine semantic distance between anatomy concepts - for instance, a brain study will be closely related to a head study (since brain will be described using a part-of relationship to the head in an ontology) resulting in prior brain studies being identified as a relevant prior to a current head study. The graphical user interface (e.g. the diagnostic viewer 208 of Fig. 3) can be provided to allow a user to further refine the list of prior studies that should be fetched from the onsite PACS system 206. For instance, radiology support staff can determine which prior studies should be fetched by selecting the relevant priors manually from a list of all priors. This list of all priors is automatically populated without the need for support staff to contact the facility to request these. The Prior Study Processor then requests the relevant priors to be fetched from the onsite PACS 206. Study details for these are communicated to the onsite gateway 220, which in turn fetches the required priors from the hospital PACS 206 via DICOM query-retrieve, and pushes to the teleradiology IT system. Prior reports are fetched via HL7 feed 224, or another suitable mechanism provided by the hospital IT system. Prior reports are then converted to a formatted text report such as an illustrative prior report 240 shown in Figure 5. The text report is converted to a DICOM object (e.g., a DICOM-compatible image) and is inserted into the current study as a new DICOM series by pushing it to the PACS gateway 220. This can now be viewed in any DICOM-compliant viewer, notably a DICOM-compliant viewer of the teleradiology reading station 204.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

## Claims

1. A non-transitory computer readable medium (26) storing instructions executable by at least one electronic processor (20) to a method (100) of selecting relevant prior reports in telemedicine, the method including:
receiving a query for prior reports (34);
selecting one or more prior reports;
re-formatting at least one of the selected prior reports; and
transmitting the re-formatted prior reports to an electronic processing device (18) operable by a medical professional.

2. The non-transitory computer readable medium (26) of claim 1, wherein the method (100) further includes:
displaying the selected prior reports (34) on an electronic processing device (18') operable by a support professional;
receiving one or more inputs, provided by the support professional, indicative of an approval of the one or more of the selected prior reports that are to be re-formatted; and
re-formatting the approved prior reports.

3. The non-transitory computer readable medium (26) of either one of claims 1 and 2, wherein re-formatting at least one of the selected prior reports (34) includes:
re-formatting the selected prior reports (34) from text to images.

4. The non-transitory computer readable medium (26) of claim 3, wherein re-formatting the selected prior reports (34) from text to images includes:
converting text of the selected prior reports to a digital imaging and communications in medicine (DICOM) image format.

5. The non-transitory computer readable medium (26) of claim 4, wherein each of the re-formatted prior reports (34) is inserted into a DICOM formatted current medical examination to be read by the medical professional as a new series inserted into the DICOM formatted current medical examination.

6. The non-transitory computer readable medium (26) of claim 5, wherein the method (100) further includes:
generating the query for the prior reports (34) based on the DICOM formatted current medical examination.

7. The non-transitory computer readable medium (26) of any one of claims 1-6, wherein the method (100) further includes:
displaying, on the electronic processing device (18) operable by a medical professional, the re-formatted prior reports (34).

8. The non-transitory computer readable medium (26) of claim 7, wherein the method (100) further includes:
performing an optical character recognition (OCR) on the displayed re-formatted prior reports (34).

9. The non-transitory computer readable medium (26) of any one of claims 1-8, wherein the method (100) further includes:
reformatting images associated with the selected prior reports (34) from an original resolution to a lower resolution; and
automatically transmitting the prior reports and the images reformatted to the lower resolution to the electronic processing device (18).

10. The non-transitory computer readable medium (26) of claim 9, wherein the method (100) further includes:
identifying one or more of the selected prior reports (34) based on one or more inputs provided a medical professional reviewing the prior reports; and
automatically transmitting the prior reports with the associated images at the original resolution to the electronic processing device (18).

11. A non-transitory computer readable medium (26) storing instructions executable by at least one electronic processor (20) to a method (100) of selecting relevant prior reports in telemedicine, the method including:
selecting one or more prior reports (34);
displaying the selected prior reports on an electronic processing device (18') operable by a support professional;
receiving one or more inputs, provided by the support professional, indicative of an approval of one or more of the selected prior reports; and
converting text of the selected prior reports to a digital imaging and communications in medicine (DICOM) image format.

12. The non-transitory computer readable medium (26) of claim 11, wherein each of the re-formatted prior reports (34) is inserted into a DICOM formatted current medical examination to be read by the medical professional as a new series inserted into the DICOM formatted current medical examination.

13. The non-transitory computer readable medium (26) of claim 12, wherein the method (100) further includes:
generating the query for the prior reports (34) based on the DICOM formatted current medical examination.

14. A method (100) of selecting relevant prior reports in telemedicine, the method including:
determining one or more prior reports (34) related to a current medical imaging examination to be read;
retrieving the one or more prior reports from a Picture Archiving and Communication System (PACS) database (32);
re-formatting the selected prior reports from text to DICOM image representations of the respective selected prior reports;
inserting the DICOM image representations of the respective selected prior reports into a DICOM representation of the current medical imaging examination to be read as new series to generate an enhanced DICOM representation of the current medical imaging examination to be read;
transmitting the enhanced DICOM representation of the current medical imaging examination to be read to an electronic processing device (18) operable by a medical professional; and
displaying, on the electronic processing device operable by a medical professional, the re-formatted prior reports.

15. The method (100) of claim 14, further including:
displaying the selected prior reports (34) on an electronic processing device (18') operable by a support professional;
receiving one or more inputs, provided by the support professional, indicative of an approval of the one or more of the selected prior reports that are to be re-formatted; and
re-formatting the approved prior reports.
